# EUROPÄISCHE PATENTSCHRIFT

(11) **EP 3 525 723 B1**
(45) Veröffentlichungstag und Bekanntmachung des Hinweises auf die Patenterteilung: **08.11.2023**
(21) Anmeldenummer: 17754604.1
(22) Anmeldetag: 28.07.2017
(51) Int. Cl.: A61F 2/14

(54) **KANÜLE ZUM FÜHREN UND ENTFALTEN EINES TRANSPLANTATS ODER IMPLANTATS**
CANNULA FOR GUIDING AND DEPLOYING A GRAFT OR IMPLANT
CANULE POUR LE GUIDAGE ET LE DÉPLOIEMENT D'UN GREFFON OU D'UN IMPLANT

(30) Priorität: 14.10.2016 DE 102016220155
(43) Veröffentlichungstag der Anmeldung: 21.08.2019
(73) Patentinhaber: Geuder AG, 69126 Heidelberg (DE)
(72) Erfinder: SAAD, Alain, 75013 Paris (FR)
(74) Vertreter: Ullrich & Naumann PartG mbB
(86) Internationale Anmeldenummer: PCT/DE2017/200076
(87) Internationale Veröffentlichungsnummer: WO 2018/068795

(56) Entgegenhaltungen:
- EP-A1- 2 601 996
- WO-A1-2009/046158
- US-A1- 2008 269 771

## Beschreibung

Die vorliegende Erfindung betrifft eine Kanüle zum Führen und Entfalten eines Transplantats oder Implantats, insbesondere zur Durchführung einer DMEK-Operation, mit einem Arbeitsbereich am und/oder nahe dem distalen Ende der Kanüle, der zum Einführen in eine vordere Augenkammer dient und derart dimensioniert ist, dass er zumindest bereichsweise in ein eingerolltes Transplantat oder Implantat einschiebbar ist, wobei die Wandung des Arbeitsbereichs am distalen Ende eine Öffnung zum Ein-/Ausströmen eines Fluids, vorzugsweise einer Flüssigkeit, aufweist.

Die Transplantation der menschlichen Hornhaut gehört zu den am häufigsten und erfolgreichsten durchgeführten transplantationschirurgischen Eingriffen. Die Transplantation der Hornhaut unter Verwendung aller ihrer Schichten (Epithel, Bowman-Membran, Stroma, Descemet-Membran und Endothelzellschicht) im Rahmen einer perforierenden Keratoplastik ist seit über 100 Jahren bekannt. Mit dieser Operationsmethode werden im Allgemeinen gute Ergebnisse erzielt, jedoch ist die Rekonvaleszenz sehr langwierig und eine Visuserholung kann üblicherweise endgültig erst nach Entfernung des zweiten Fadens erreicht werden. Dies kann eine Zeitspanne von bis zu 18 Monaten nach dem Eingriff ausmachen.

Gut die Hälfte aller Hornhauttransplantationen wird wegen Erkrankungen des Hornhautendothels durchgeführt. Für diese Erkrankungen würde sich prinzipiell ein schichtspezifischer Ersatz der Hornhaut eignen. Geeignete Techniken hierfür liegen mittlerweile vor. Durch die Transplantation der Descemet'schen Membran mit einer anhängenden Stromalamelle (DSAEK) kann seit einigen Jahren eine im Vergleich mit der perforierenden Keratoplastik viel schnellere Visuserholung und demzufolge eine bessere Patientenzufriedenheit erreicht werden.

Zur Therapie bei Erkrankungen der Hornhaut, welche das Hornhautendothel betreffen, ist des Weiteren eine neuartige, spezielle Form der Hornhauttransplantation beschrieben worden. Dabei handelt es sich um die Descemet Membrane Endothelial Keratoplasty (DMEK). Im Zuge eines solchen Eingriffs können die erkrankten Endothel-Zellen einschließlich der darunter liegenden Descemet-Membran entfernt und durch eine Descemet-Membran mit gesundem Hornhautendothel eines Spenders ersetzt werden. Auf eine perforierende Keratoplastik kann dabei verzichtet werden, vielmehr lässt sich das Transplantat durch eine vergleichsweise kleine Inzision in die vordere Augenkammer transferieren und durch sehr vorsichtige Manipulation ausbreiten. Danach wird das Membran-Präparat mittels Luftzugabe am hinteren Stroma fixiert.

Eine erfolgreiche DMEK-Operation ermöglicht dem Patienten eine sehr schnelle visuelle Rehabilitation. Es ist davon auszugehen, dass der Patient schon nach ca. zwei Monaten nach dem Eingriff eine volle Sehschärfe erreicht.

Allgemein sind eine ganze Reihe chirurgischer und insbesondere ophthalmologischer Eingriffe bekannt oder denkbar, bei denen ein Transplantat oder ein Implantat bereitgestellt und in den lebenden Körper eingebracht wird. Dabei ist es im Sinne einer raschen Rekonvaleszenz des Patienten - wie oben anhand der DMEK-Operation beispielhaft ausgeführt - vorteilhaft, ein Transplantat oder Implantat durch eine Inzision begrenzter, und insbesondere möglichst kleiner Größe in den lebenden Körper einzubringen.

Wird eine geeignete Vorrichtung zum Einbringen eines Transplantats oder eines Implantats verwendet, stellt sich jedoch das Problem, das Transplantat oder Implantat ohne jede Beschädigung und ggf. in einer konkreten Lage bzw. Position in dieser Vorrichtung bereitzustellen. Insbesondere für den Fall, dass die Vorrichtung zum Einbringen eines Transplantats in eine sehr kleine Inzision im Körper des Patienten geeignet ist, kann das Transplantat aufgrund der zwangsläufig geringen Ausmaße einer solchen Vorrichtung bereits beim Bereitstellen in der Vorrichtung beschädigt oder zerstört werden. Gegebenenfalls ist ein Bereitstellen des Transplantats in der Vorrichtung aufgrund der geringen Abmessungen der Vorrichtung bereits anfangs unmöglich.

Aus der DE 10 2010 051 458 A1 und WO 2016/095884 A1 sind zwei alternative Beispiele zum sicheren und einfachen Bereitstellen und zum Einbringen eines Transplantats oder eines Implantats in den lebenden Körper, insbesondere für ophthalmologische Eingriffe, bekannt. Die beschriebenen Vorrichtungen weisen eine Kartusche oder Hülse mit zwei gegenüberliegenden, endseitigen Durchgängen auf, wobei diese Kartuschen oder Hülsen in besonderer Weise geeignet sind, ein Transplantat oder Implantat durch eine sehr kleine Inzision in den Körper eines Patienten einzubringen, wie dies beispielsweise bei der zuvor beschriebenen ophthalmologischen Anwendung erforderlich ist.

Zum Bereitstellen und Einbringen ist das Transplantat oder Implantat in eine wie oben beschriebene Kartusche aufgenommen und darin zum Einbringen in die vordere Augenkammer durch eine verhältnismäßig kleine Inzision aufgerollt. Die Kartusche ist bspw. auf eine Spritze aufsteckbar, so dass durch Betätigung des Kolbens der Spritze das Transplantat oder Implantat zusammen mit der in der Spritze befindlichen Flüssigkeit, meist Kochsalzlösung, in den Körper des Patienten transferiert werden kann. In der vorderen Augenkammer soll das Transplantat oder Implantat positioniert und entrollt werden. Dies lässt sich nicht ohne Weiteres, zumindest nicht mittels der voranstehend beschriebenen Vorrichtung zum Bereitstellen und Einbringen eines Implantats bzw. Transplantats, bewerkstelligen.

In der Praxis werden zum Führen und Entfalten eines Transplantats oder Implantats Entfaltungskanülen verwendet. Diese Kanülen werden durch eine zweite, der ersten Inzision zur Einführung des Transplantats bzw. Implantats im Wesentlichen gegenüberliegenden Inzision ebenfalls in die vordere Augenkammer eingeführt. Die Kanüle bzw. das distale Ende der Kanüle bildet einen Arbeitsbereich, der derart dimensioniert ist, dass er zumindest bereichsweise in ein eingerolltes Transplantat oder Implantat einschiebbar ist und zur Positionierung und Entfaltung somit "abholen", d.h. von der Kartusche übernehmen kann.

Aus der Praxis bekannte Entfaltungskanülen sind an eine druckerzeugende Vorrichtung zur Bereitstellung eines Fluids gekoppelt, beispielsweise an eine Spritze oder Pumpe, und weisen eine einzige Öffnung am distalen Ende der Kanüle auf, um das Transplantat oder das Implantat zu entrollen. Dabei entsteht jedoch ein derart starker Fluidstrom/Jet-Stream, dass das Transplantat bzw. Implantat unkontrolliert von der Mitte der vorderen Augenkammer weggetrieben wird. Dies kann zu einer Fehlpositionierung des Transplantats oder Implantats führen, was eine ehebliche zeitliche Verzögerung des Eingriffs, eine zusätzliche Inzision oder zumindest die Einführung weiterer Werkzeug zur Positionierung, eine Beschädigung oder gar einen Verlust des Transplantats bzw. Implantats zur Folge haben kann. In der Praxis wird die Positionierung und das Entfaltens durch vorsichtiges "Klopfen" auf die Cornea unterstützt. Dies ist jedoch eine nicht unkritische und wenig zufriedenstellende Methode.

Der vorliegenden Erfindung liegt daher die Aufgabe zugrunde, eine Kanüle der gattungsbildenden Art derart auszugestalten und weiterzubilden, dass eine kontrollierte und sichere Entfaltung realisierbar ist.

Die voranstehende Aufgabe ist erfindungsgemäß durch die Merkmale des Anspruchs 1 gelöst.

Eine Kanüle wie im Oberbegriff von Anspruch 1 angegeben, ist aus der Druckschrift US 2008/269771 bekannt.

In erfindungsgemäßer Weise ist erkannt worden, dass mehrere Fluidausgänge an unterschiedlichen Stellen und Fluidströme in unterschiedliche Richtungen ein kontrolliertes und sicheres Entrollen des Transplantats oder Implantats begünstigen. Dies wird dadurch realisiert, dass die Wandung des Arbeitsbereichs - zusätzlich zu der Öffnung am distalen Ende - mindestens eine, vorzugsweise zwei seitliche Öffnungen aufweist, durch die ein Fluidstrom in im Wesentlichen radiale Richtung der Kanüle ausströmt. Der Strömungsaustritt wird somit auf mehrere Öffnungen entlang des Arbeitsbereichs der Kanüle verteilt. Ein derart starker Fluidstrom/Jet-Stream, der das Transplantat oder Implantat unkontrolliert von der Mitte der vorderen Augenkammer wegtreibt, ist somit vermeidbar. Eine Realisierung ausschließlich seitlicher Öffnungen, d.h. ohne eine Öffnung am distalen Ende der Kanüle, ist ebenso denkbar.

Folglich ist mit der erfindungsgemäßen Kanüle eine Kanüle realisiert, die ein kontrolliertes und sicheres Entfalten ermöglicht.

Um die Strömungsverteilung über die gesamte Länge des Arbeitsbereichs und dadurch das Entfalten des Transplantats oder Implantats abermals zu optimieren, ist es von besonderem Vorteil, wenn die Dimensionierung und/oder die Positionierung der Öffnungen derart ausgelegt ist/sind, dass der Volumenstrom des Fluids in den Öffnungen, zumindest jedoch in den seitlichen Öffnungen nahezu identisch ist. Durch gleichmäßige Verteilung der Volumenströme über alle Öffnungen lässt sich das Transplantat oder das Implantat in der vorderen Augenkammer mittig positionieren und kontrolliert und sicher entfalten.

Durch die Realisierung paarweiser, seitlicher Öffnungen, die vorzugsweise einander gegenüberliegend vorgesehen sind, lässt sich eine genaue Positionierung sowie ein kontrolliertes und sicheres Entrollen des Transplantats oder Implantats weiter verbessern. Denkbar sind auch mehr als drei sich gegenüberliegende Öffnungen auf einer Höhe entlang des Arbeitsbereichs, die jeweils einen Fluidstrom in unterschiedliche Richtungen gewährleisten.

In weiter vorteilhafter Weise sind die seitlichen Öffnungen oder die Paare oder Gruppen von gegenüberliegenden, d.h. auf einer Höhe entlang des Arbeitsbereichs liegenden Öffnungen äquidistant zueinander angeordnet. Dies begünstigt abermals eine vorteilhafte Strömungsverteilung entlang der gesamten Länge des Arbeitsbereichs und hat positive Auswirkungen auf ein sicheres Entrollen des Transplantats oder Implantats.

Zur Gewährleistung eines gleichmäßigen Volumenstroms sind die Öffnungen in vorteilhafter Weise unterschiedlich groß dimensioniert und weisen insbesondere unterschiedliche Durchmesser auf.

Hierzu ist es von besonderem Vorteil, wenn die Öffnung am distalen Ende der Kanüle kleiner dimensioniert ist als die seitlichen Öffnungen.

Ein idealer Druckaufbau entsteht dann bzw. ergibt sich der Effekt eines gleichmäßigen Volumenstroms dann besonders gut, wenn in vorteilhafter Weise die Öffnungen vom distalen Ende der Kanüle in Richtung des proximalen Endes der Kanüle größer werden. In anderen Worten sind die Öffnungen, je weiter sie vom distalen Ende der Kanüle entfernt liegen, größer.

Grundsätzlich ist es denkbar, das die Öffnungen, zumindest jedoch die seitlichen Öffnungen, im Wesentlichen die gleiche Form aufweisen. Bevorzugt ist eine im Wesentlichen runde Form der Öffnungen. Andere Formen, wie bspw. eckige oder ovale Formen sind ebenso denkbar.

Durch sich im Querschnitt nach außen vorzugsweise konisch erweiternde Öffnungen kann sich der Fluidstrom in vorteilhafter Weise abermals in mehrere Richtungen verteilen und kann weiterhin ein kontrolliertes und schonendes Entrollen des Transplantats oder Implantats begünstigt werden.

Um ein zumindest bereichsweises Einschieben der Kanüle bzw. des Arbeitsbereichs der Kanüle in das zusammengerollte Transplantat oder Implantat zu gewährleisten, verjüngt sich in vorteilhafter Weise der Arbeitsbereich der Kanüle am distalen Ende. Der Arbeitsbereich bzw. das distale Ende ist vorzugsweise derart ausgeführt, dass es nicht nur in das eingerollte Transplantat oder Implantat einschiebbar ist, sondern das Transplantat oder Implantat von der Kartusche "abholen", d.h. aus der Kartusche herausführen und in der vorderen Augenkammer mittig positionieren kann. Insofern sollte der Arbeitsbereich entsprechend dem Innendurchmesser der Kartusche bzw. des aufgerollten Transplantats oder Implantats weder zu dick noch zu dünn ausgeführt sein. Denkbar ist auch eine derartige Ausführung von Kartusche und/oder Kanüle, so dass die Kanüle an die Kartusche andockbar ist.

Zur Realisierung eines Fluidstroms in der Kanüle bzw. in dessen Arbeitsbereich bestehen zwei Möglichkeiten. Bevorzugt ist ein Fluid über das proximale Ende der Kanüle in den Arbeitsbereich einführbar. Hierzu ist die Kanüle an ihrem proximalen Ende an eine Vorrichtung zur Bereitstellung eines Fluids unter Druck, beispielsweise über eine an die Kanüle gekoppelte Spritze, gekoppelt. Alternativ oder zusätzlich ist es denkbar, dass ein Fluid bzw. Flüssigkeit aus der das Transplantat oder Implantat bereitstellenden und einführenden Vorrichtung bzw. Kartusche durch das eingerollte Transplantat oder Implantat hindurch über das distale Ende der Kanüle bzw. die Öffnung am distalen Ende der Kanüle in den Arbeitsbereich eintritt. In diesem Fall wäre eine Ankopplung der Kanüle an eine ein Fluid bereitstellende Vorrichtung nicht notwendig.

Des Weiteren sei an dieser Stelle angemerkt, dass die Kanüle aus Metall, Glas oder Kunststoff hergestellt sein kann. Auch andere, für die Chirurgie geeignete Materialien sind denkbar. Ferner kann die erfindungsgemäße Kanüle in einer sterilen Verpackung bereitgestellt und als Einwegprodukt ausgeführt sein.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche und andererseits auf die nachfolgende Erläuterung eines bevorzugten Ausführungsbeispiels der erfindungsgemäßen Vorrichtung zu verweisen. In Verbindung mit der Erläuterung des bevorzugten Ausführungsbeispiels anhand der Zeichnung werden auch im Allgemeinen bevorzugte Ausgestaltungen und Weiterbildungen der Lehre erläutert. In der Zeichnung zeigen
- Fig. 1: in einer schematischen Ansicht ein Ausführungsbeispiel einer erfindungsgemäßen Kanüle bei der Durchführung einer DMEK-Operation, und
- Fig. 2: in einer schematischen Ansicht eine Vergrößerung des Ausführungsbeispiels aus Fig. 1.

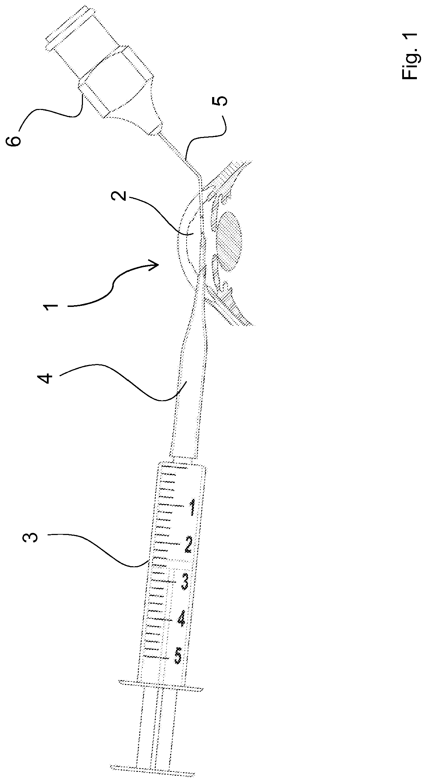

Fig. 1 zeigt in einer schematischen Ansicht eine DMEK-Operation am menschlichen Auge. Im Konkreten ist ein Abschnitt eines menschlichen Auges 1 gezeigt, wobei von links eine Vorrichtung zum Einbringen eines Transplantats und von rechts eine Vorrichtung zum Führen und Positionieren eines Transplantats in die vordere Augenkammer 2 eingreifen. Die Vorrichtung zum Einbringen eines Transplantats umfasst eine Spritze 3 mit einer an der Düse der Spritze 3 aufgesteckten Kartusche 4, deren Spitze durch eine kleine Inzision in die vordere Augenkammer 2 ragt. Die Vorrichtung zum Führen und Entfalten des Transplantats umfasst eine Kanüle 5, die an ihrem distalen Ende ebenfalls durch eine kleine Inzision in die vordere Augenkammer 2 hineinragt und an ihrem proximalen Ende an einer Vorrichtung zur Bereitstellung einer Flüssigkeit unter Druck, insbesondere einer Handpumpe 6, angekoppelt ist.

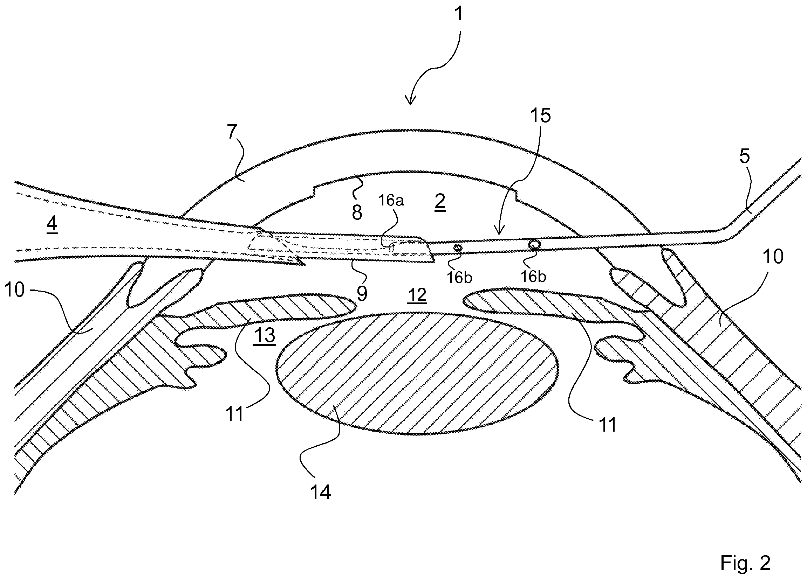

Weitere Einzelheiten sind vergrößert in Fig. 2 gezeigt. Fig. 2 zeigt einen Augenabschnitt eines menschlichen Auges 1 mit einer Cornea 7, wobei die Descemet-Membran 8 zur Ersetzung durch ein Transplantat 9 endothelseitig entfernt ist, einer vorderen Augenkammer 2, einer Lederhaut 10, einer Iris 11, einer Pupille 12, einer hinteren Augenkammer 13 und einer Linse 14.

Linksseitig ragt eine Kanüle 4 durch eine kleine Inzision in die vordere Augenkammer 2, die zum Einbringen des Transplantats 9 dient. Das Transplantat 9 ist bei Bereitstellung in der Kartusche 4 eingerollt und durch Betätigung des Kolbens der Spritze 3 im zusammengerolltem Zustand aus der Kartusche 4 hinaus und in die vordere Augenkammer transferierbar.

Rechtsseitig ragt durch eine zweite kleine Inzision eine Kanüle 5 in der Kartusche 4 entgegengesetzte Richtung in die vordere Augenkammer 2. Die Kanüle 5 dient zum Führen und Entfalten des eingerollten Transplantats 9 und weist einen Arbeitsbereich 15 am und nahe dem distalen Ende der Kanüle 5 auf. Der Arbeitsbereich 15 dient dem Einführen in die vordere Augenkammer 2 und ist derart dimensioniert, dass er zumindest bereichsweise in das eingerollte Transplantat 9 einschiebbar ist und das Implantat "abholen", d.h. vollständig aus der Kartusche 4 entnehmen und positionieren kann. Durch Bewegung der Kanüle ist das Transplantat mittig in die vordere Augenkammer positionierbar.

Die Wandung des Arbeitsbereichs 15 weist am distalen Ende der Kanüle 5 eine Öffnung 16a auf. Weitere Öffnungen 16b sind seitlich an der Wandung des Arbeitsbereichs vorgesehen. Die Öffnungen 16a, 16b dienen dem kontrollierten Entfalten des Transplantats 9 durch einen Flüssigkeitsstrom von innerhalb nach außerhalb der Kanüle in das Kammerwasser. Die Öffnungen 16a, 16b sind derart dimensioniert und positioniert, dass der Volumenstrom der Flüssigkeit in den Öffnungen nahezu identisch ist. Die vier seitlichen Öffnungen 16b sind paarweise und einander gegenüberliegend vorgesehen, wodurch ein ungewolltes wegtreiben des Transplantats 9 gewährleistet ist. Die Öffnungen 16a, 16b bzw. die Öffnung 16a am distalen Ende und die Paare von gegenüberliegenden Öffnungen 16b sind äquidistant zueinander angeordnet. Die Öffnungen 16a, 16b sind weiterhin unterschiedlich groß dimensioniert, weisen im Konkreten unterschiedliche Durchmesser auf. Die Öffnung 16a am distalen Ende der Kanüle 5 ist kleiner dimensioniert als die seitlichen Öffnungen 16b. Je weiter die seitlichen Öffnungen 16b vom distalen Ende der Kanüle 5 entfernt sind, desto größer ist ihr Durchmesser. Idealerweise betragen die Durchmesser der paarweisen Öffnungen 16b 0,1, 0,15 und 0,2 mm. Die unterschiedlichen Durchmesser der Öffnungen 16a, 16b gewährleisten einen gleichmäßigen Volumenstrom. Dadurch wird die Strömungsverteilung entlang der gesamten Kanüle 5 bzw. des Arbeitsbereichs 15 optimiert. Die Öffnungen 16a, 16b sind im Wesentlichen rund, wobei sich ihr Querschnitt nach außen hin konisch erweitert. Die Flüssigkeitsströme aus den Öffnungen 16a, 16b erfolgen aufgrund ihrer Dimensionierung, Form und Positionierung in unterschiedliche Richtungen, so dass das Transplantat in idealer Weise kontrolliert und sicher entfaltbar ist.

Zum Druckaufbau im Inneren der Kanüle 5 bzw. des Arbeitsbereichs 15, um ausreichend Volumenstrom in allen Öffnungen 16a, 16b zu realisieren, ist der Innendurchmesser des Arbeitsbereichs 15 verhältnismäßig klein. Am distalen Ende der Kanüle 5 ist der Arbeitsbereich 15 verjüngt, so dass er optimal und sicher in das eingerollte Transplantat einführbar ist.

Durch Druck aus der Spritze 3 wird das Transplantat 9 aus der Kartusche 4 in die vordere Augenkammer 2 hinausbefördert und gleichzeitig durch den Arbeitsbereich 15 der Kanüle 5 "abgeholt". Ist der Arbeitsbereich 15 ausreichend weit in das eingerollte Transplantat 9 gerutscht oder eingeführt, wird das Transplantat 9 durch Bewegung der Kanüle 5 in die Mitte der vorderen Augenkammer 2 positioniert und durch den aus den Öffnungen 16a, 16b austretenden Flüssigkeitsstrom kontrolliert und sicher entfaltet.

Zur Vermeidung von Wiederholungen sei in Bezug auf Merkmale, die sich den Figuren nicht entnehmen lassen, auf den allgemeinen Teil der Beschreibung verwiesen.

Schließlich sei ausdrücklich darauf hingewiesen, dass das voranstehend beschriebene Ausführungsbeispiel lediglich zur Erörterung der beanspruchten Lehre dient, diese jedoch nicht auf das Ausführungsbeispiel einschränkt.

### Bezugszeichenliste

- 1: Ausschnitt eines menschlichen Auges
- 2: Vordere Augenkammer
- 3: Spritze
- 4: Kartusche zum Einbringen eines Transplantats
- 5: Kanüle zum Führen und Entfalten eines Transplantats
- 6: Vorrichtung zur Bereitstellung einer Flüssigkeit /Handpumpe
- 7: Cornea
- 8: Descemet-Membran, Endothel
- 9: Transplantat
- 10: Lederhaut
- 11: Iris
- 12: Pupille
- 13: Hintere Augenkammer
- 14: Linse
- 15: Arbeitsbereich der Kanüle
- 16a: Vordere Öffnung am distalen Ende der Kanüle
- 16b: Seitliche Öffnungen der Kanüle

## Patentansprüche

1. Kanüle (5) zum Führen und Entfalten eines Hornhaut-Transplantats (9) oder - Implantats, insbesondere zur Durchführung einer sog. Descemet Membrane Endothelial Keratoplasty (DMEK) -Operation, mit einem Arbeitsbereich (15) am und/oder nahe dem distalen Ende der Kanüle (5), der zum Einführen in eine vordere Augenkammer (2) dient und derart dimensioniert ist, dass er zumindest bereichsweise in ein eingerolltes Hornhaut-Transplantat (9) oder -Implantat einschiebbar ist, wobei die Wandung des Arbeitsbereichs (15) am distalen Ende eine Öffnung (16a) und mindestens eine, vorzugsweise zwei seitliche Öffnungen (16b) zum Ein-/Ausströmen eines Fluids, vorzugsweise einer Flüssigkeit, aufweist,
**dadurchgekennzeichnet**, dass die Öffnung (16a) am distalen Ende im Wesentlichen mittig in Längsrichtung der Kanüle angeordnet ist und die Dimensionierung und/oder die Positionierung der Öffnungen (16a, 16b) derart ausgelegt ist, dass der Volumenstrom des Fluids zumindest in den seitlichen Öffnungen (16b) nahezu identisch ist, wobei die Öffnungen (16a, 16b) unterschiedlich groß dimensioniert sind, insbesondere unterschiedliche Durchmesser aufweisen.

2. Vorrichtung nach Anspruch 1, **dadurch gekennzeichnet, dass** die seitlichen Öffnungen (16b) paarweise, vorzugsweise einander gegenüber liegend vorgesehen sind.

3. Vorrichtung nach einem der Ansprüche 1 bis 2, **dadurch gekennzeichnet, dass** zumindest die seitlichen Öffnungen (16a, 16b) oder die Paare von gegenüber liegenden Öffnungen (16b) äquidistant zueinander angeordnet sind.

4. Vorrichtung nach einem der Ansprüche 1 bis 3, **dadurch gekennzeichnet, dass** die Öffnung (16a) am distalen Ende der Kanüle (5) kleiner dimensioniert ist, als die seitlichen Öffnungen (16b).

5. Vorrichtung nach einem der Ansprüche 1 bis 4, **dadurch gekennzeichnet, dass** die Öffnungen (16a, 16b), je weiter sie vom distalen Ende der Kanüle (5) entfernen sind, größer sind.

6. Vorrichtung nach einem der Ansprüche 1 bis 5, **dadurch gekennzeichnet, dass** zumindest die seitlichen Öffnungen (16a, 16b) im Wesentlichen die gleiche Form oder unterschiedliche Formen aufweisen, vorzugsweise im Wesentlichen rund sind.

7. Vorrichtung nach einem der Ansprüche 1 bis 6, **dadurch gekennzeichnet, dass** sich der Querschnitt der Öffnungen (16a, 16b) vorzugsweise konisch nach außen erweitert.

8. Vorrichtung nach einem der Ansprüche 1 bis 7, **dadurch gekennzeichnet, dass** sich der Arbeitsbereich (15) am distalen Ende der Kanüle (5) verjüngt.

9. Vorrichtung nach einem der Ansprüche 1 bis 8, **dadurch gekennzeichnet, dass** die Strömung des Fluids über das proximale Ende und/oder über das distale Ende der Kanüle (5) in den Arbeitsbereich (15) eintritt.

10. Vorrichtung nach einem der Ansprüche 1 bis 9, **dadurch gekennzeichnet, dass** die Kanüle (5) an eine Vorrichtung zur Bereitstellung eines Fluids (6), vorzugsweise eine Spritze oder Pumpe ankoppelbar ist.

## Claims

1. Cannula for guiding and deploying a cornea transplant (9) or implant, in particular for carrying out a so-called Descemet membrane endothelial keratoplasty (DMEK) operation, having on and/or close to the distal end of the cannula (5) a working region (15) which is used for introduction into a front eye chamber (2) and which is sized in such a manner that it can be at least partially pushed into a rolled-up cornea transplant (9) or implant, wherein the wall of the working region (15) has at the distal end an opening (16a) and at least one, preferably two lateral openings (16b) for influx/discharge of a fluid, preferably a liquid, **characterised in that** the opening (16a) at the distal end is arranged substantially centrally in the longitudinal direction of the cannula and the sizing and/or the positioning of the openings (16a, 16b) is configured in such a manner that the volume flow of the fluid at least in the lateral openings (16b) is almost identical, wherein the openings (16a, 16b) have different dimensions, in particular have different diameters.

2. Apparatus according to claim 1, **characterised in that** the lateral openings (16b) are provided in pairs, preferably opposite each other.

3. Apparatus according to either claim 1 or claim 2, **characterised in that** at least the lateral openings (16a, 16b) or the pairs of opposing openings (16b) are arranged to be equidistant from each other.

4. Apparatus according to any one of claims 1 to 3, **characterised in that** the opening (16a) is sized to be smaller at the distal end of the cannula (5) than the lateral openings (16b).

5. Apparatus according to any one of claims 1 to 4, **characterised in that** the openings (16a, 16b) are larger the further they are away from the distal end of the cannula (5).

6. Apparatus according to any one of claims 1 to 5, **characterised in that** at least the lateral openings (16a, 16b) have substantially the same shape or different shapes, preferably are substantially round.

7. Apparatus according to any one of claims 1 to 6, **characterised in that** the cross-section of the openings (16a, 16b) preferably expands outwards in a conical manner.

8. Apparatus according to any one of claims 1 to 7, **characterised in that** the working region (15) tapers at the distal end of the cannula (5).

9. Apparatus according to any one of claims 1 to 8, **characterised in that** the flow of the fluid via the proximal end and/or via the distal end of the cannula (5) enters the working region (15).

10. Apparatus according to any one of claims 1 to 9, **characterised in that** the cannula (5) can be coupled to an apparatus for providing a fluid (6), preferably a syringe or pump.

## Revendications

1. Canule (5) pour le guidage et le déploiement d'un transplant ou implant de cornée (9) ou implant, plus particulièrement pour l'exécution de ce qu'on appelle une opération Dscemet Membrane Endothelial Heratoplasty (DMEK), avec une zone de travail (15) sur et/ou proche de l'extrémité distale de la canule (5), qui permet son introduction dans une chambre oculaire avant (2) et est dimensionnée de façon à ce qu'elle puisse être insérée, au moins à certains endroits, dans un transplant de cornée (9) ou implant enroulé, dans lequel la paroi de la zone de travail (15) présente, à l'extrémité distale, une ouverture (16a) et au moins une, de préférence deux ouvertures latérales (16b), pour l'entrée/sortie d'un fluide, de préférence d'un liquide,
**caractérisé en ce que** l'ouverture (16a) est disposée au niveau de l'extrémité distale, de manière globalement centrale dans la direction longitudinale de la canule et le dimensionnement et/ou le positionnement des ouvertures (16a, 16b) est conçu de façon à ce que le débit volumique du fluide soit presque identique dans les ouvertures latérales (16b), dans lequel les ouvertures (16a, 16b) présentent des dimensions différentes, plus particulièrement des diamètres différents.

2. Dispositif selon la revendication 1, **caractérisé en ce que** les ouvertures latérales (16b) sont disposées par paires, de préférence de manière superposée.

3. Dispositif selon l'une des revendications 1 à 2, **caractérisé en ce qu'**au moins les ouvertures latérales (16a, 16b) ou les paires d'ouvertures opposées (16b) sont disposées de manière équidistante entre elles.

4. Dispositif selon l'une des revendications 1 à 3, **caractérisé en ce que** l'ouverture (16a) présente, à l'extrémité distale de la canule (5), une dimension plus petite que les ouvertures latérales (16b).

5. Dispositif selon l'une des revendications 1 à 4, **caractérisé en ce que** les ouvertures (16a, 16b) sont d'autant plus grandes qu'elles sont éloignées de l'extrémité distale de la canule (5).

6. Dispositif selon l'une des revendications 1 à 5, **caractérisé en ce qu'**au moins les ouvertures latérales (16a, 16b) présentent globalement la même forme ou des formes différentes, de préférence sont globalement rondes.

7. Dispositif selon l'une des revendications 1 à 6, **caractérisé en ce que** la section transversale des ouvertures (16a, 16b) s'élargit de préférence de manière conique vers l'extérieur.

8. Dispositif selon l'une des revendications 1 à 7, **caractérisé en ce que** la zone de travail (15) se rétrécit à l'extrémité distale de la canule (5).

9. Dispositif selon l'une des revendications 1 à 8, **caractérisé en ce que** l'écoulement du fluide entre par l'extrémité proximale et/ou par l'extrémité distale de la canule (5) dans la zone de travail (15).

10. Dispositif selon l'une des revendications 1 à 9, **caractérisé en ce que** la canule (5) peut être couplée à un dispositif de mise à disposition d'un fluide (6), de préférence une seringue ou une pompe.
